# EUROPEAN PATENT APPLICATION

(11) **EP 4 556 016 A1**
(43) Date of publication of application: **21.05.2025**
(21) Application number: 24199515.8
(22) Date of filing: 10.09.2024
(51) Int. Cl.: A61K 36/282, A61K 36/534, A61K 36/539, A61K 36/808, A61K 36/815, A61K 36/8964, A61K 35/64, A61P 11/00

(54) **TRADITIONAL CHINESE MEDICINE COMPOSITION FOR TREATING PEDIATRIC EXOGENOUS WIND-HEAT AND PREPARATION THEREFOR**

(30) Priority: 16.11.2023 CN 202311530281
(71) Applicant: Jiuhua Huayuan Pharmaceutical Co., Ltd., Chuzhou (CN)
(72) Inventor: Guan, Yueqin, Chuzhou (CN); An, Xiaoxian, Chuzhou (CN); Zhang, Jiquan, Chuzhou (CN); Chen, Lingwu, Chuzhou (CN); Liu, Yadong, Chuzhou (CN); Wang, Xin, Chuzhou (CN); Luo, Xuefeng, Chuzhou (CN); Ding, Jianya, Chuzhou (CN); Zhang, Gusi, Chuzhou (CN)
(74) Representative: Petraz, Gilberto Luigi

(57) **Abstract**

A traditional Chinese medicine composition for treating pediatric exogenous wind-heat and a preparation method thereof are provided and relate to the field of traditional Chinese medicine technology. The traditional Chinese medicine composition for treating the pediatric exogenous wind-heat includes the following raw materials: Menthae Haplocalycis Herba, Bombyx Batryticatus, Gypsum Fibrosum, Anemarrhenae Rhizoma, Scutellariae Radix, Artemisiae Annuae Herba, Scrophulariae Radix, and Lycii Cortex. This traditional Chinese medicine composition can treat pediatric exogenous wind-heat invading the exterior pattern can significantly improve the clinical symptoms and signs of the pediatric patient and enhance the body's immunity, with remarkable effectiveness and high safety, thus being worthy of clinical promotion and application.

## Description

### TECHNICAL FIELD

The present application relates to the technical field of traditional Chinese medicine, more particularly to a traditional Chinese medicine composition for treating pediatric exogenous wind-heat and a preparation method therefor.

### BACKGROUND

Commonly known as a common cold, a cold disorder is an infectious inflammation of the upper respiratory tract caused by viruses or bacteria. It is highly contagious and can occur in all seasons, but is more common in winter and spring. Traditional Chinese medicine believes that when the climate changes suddenly, the cold and heat are abnormal, or the wind blows when going out, the internal and external functions of the human body are weakened, and the wind-cold and wind-heat take advantage of the deficiency to invade, invading the lung and defense qi, and the defense surface is not solid and causes disease. The main symptoms are fever, nasal congestion, runny nose, sore throat, and headache, and sometimes joint pain and general discomfort. Pediatric wind-heat cold is a common disease. Because children have special physiological and disease characteristics, the various pains and diseases caused by children's illnesses cause adverse harm to children's bodies. Although most children's diseases are not difficult to diagnose clinically, the implementation for the treatment is more difficult. There are many pharmaceutical preparations on the market to treat children's colds, including Western medicine and Chinese medicine. Western medicine has serious side effects, and Chinese medicine has a worse smell. Children are weak, so when using medication, attention should be paid to both the efficacy and the child's tolerance. Therefore, how to develop a traditional Chinese medicine composition with significant efficacy on children has become a realistic issue.

### SUMMARY

It is an objective of the present application to provide a traditional Chinese medicine composition for treating pediatric exogenous wind-heat, which has significant efficacy for pediatric patients.

It is another objective of the present application to provide two preparation methods for the traditional Chinese medicine composition for treating pediatric exogenous wind-heat, to produce this highly effective composition.

To address the above technical problems, the following technical solutions are adopted.

In a first aspect, embodiments of the present application provides a traditional Chinese medicine composition for treating pediatric exogenous wind-heat, which comprises the following raw materials: a Menthae Haplocalycis Herba, a Bombyx Batryticatus, a Gypsum Fibrosum, an Anemarrhenae Rhizoma, a Scutellariae Radix, an Artemisiae Annuae Herba, a Scrophulariae Radix, and a Lycii Cortex.

In a second aspect, embodiments of the present application provides a preparation method for the traditional Chinese medicine composition for treating the pediatric exogenous wind-heat. The preparation method comprises the following steps:
collecting and mixing the raw materials to obtain a first mixture;
submerging the first mixture in water, soaking and decocting the first mixture, and filtering to obtain a primary filtrate and a filtration residue; mixing the filtration residue with water, decocting, and filtering to obtain a secondary filtrate; and combining the primary filtrate and the secondary filtrate to obtain an extract;
concentrating the extract to obtain a solid extract paste; and
screening the solid extract paste, and mixing a screened solid extract paste with an excipient to form granules, whereby obtaining the traditional Chinese medicine composition for treating the pediatric exogenous wind-heat.

In a third aspect, a preparation method for the traditional Chinese medicine composition for treating the pediatric exogenous wind-heat comprises the following steps:
collecting and mixing the raw materials to obtain a second mixture;
submerging the second mixture in water, heating until reaching a boiling state, and decocting the second mixture at the boiling state, then filtering to obtain a decoction liquid and a decoction residue; mixing the decoction residue with water, heating and refluxing to obtain a reflux liquid, and mixing the decoction liquid with the reflux liquid, concentrating a resulting mixed liquid to obtain a concentrated liquid; and
spray drying the concentrated liquid and mixing the same with an excipient to form granules , and screening to obtain the traditional Chinese medicine composition for treating the pediatric exogenous wind-heat.

Compared with existing technology, advantages or beneficial effects of the traditional Chinese medicine composition for treating pediatric exogenous wind-heat and the preparation method therefor according to embodiments of the present application are summarized as follows:

The researchers of the present application believe that pediatric upper respiratory infections fall under the category of "colds" or "wind-cold" in traditional Chinese medicine (TCM). Since the lung governs the skin and the pores, and children's organs are delicate with weak lung defense functions, when they are exposed to the wind-heat, or the wind-cold enters the interior and transforms into heat, then the defensive surface is out of harmony and the lung qi fail to disperse properly. This leads to symptoms of defensive surface and the upper jiao of pulmonary system, such as fever, nasal congestion, runny nose, cough, and headache.

Based on the above reasons, the present application uses the Menthae Haplocalycis Herba, the Bombyx Batryticatus, and the Artemisiae Annuae Herba which are clear and refreshing flavored, gently cooling and dispersing, and are effective in releasing Wei-defence phase pattern; as well as the Gypsum Fibrosum, the Anemarrhenae Rhizoma, and the Scutellariae Radix, which are bitter and cold flavored, and cold and cool-natured, and are effective in clearing heat from the qi level; added with Scrophulariae Radix and the Lycii Cortex, which help nourishing yin and generating fluids, draining fire and detoxifying, preventing heat from damaging yin, and have the purpose of "for every measure of body fluids preserved, there is a measure of vitality maintained". In this way, external wind is dispersed, heat and toxins are cleared, yin fluids are preserved, and fever is rapidly reduced to aid in the pediatric patient's recovery. The combination of these components have the effect of dispersing wind and dissipating heat, clearing heat and detoxifying, generating fluids and protecting yin, function in treating the pattern involving both Wei-defence and qi phases, and treating both the surface and interior conditions, and are primarily used to removing wind and clearing heat, and detoxifying and protecting yin, so as to treating pediatric exogenous wind-heat and the pattern involving both Wei-defence and qi phases, with symptoms such as persistent high fever, sweating or scant sweating, nasal congestion, runny nose, red and swollen throat, yellow urine, dry stool, red tongue with thin white or thin yellow coating, and slippery rapid pulse, and pediatric upper respiratory tract infections with the above syndromes.

In conclusion, the traditional Chinese medicine composition provided in the present application can effectively treat pediatric wind-heat exterior conditions, significantly improve clinical symptoms and signs in pediatric patient, enhance immune function, and has a notable therapeutic effect with high safety, making it suitable for clinical promotion.

The present application also provides two preparation methods for the traditional Chinese medicine composition, which maximize the extraction of beneficial substances from the ingredients and efficiently and conveniently produce the traditional Chinese medicine composition, which facilitates the bulk production.

### DETAILED DESCRIPTION OF THE EMBODIMENTS

To clarify the objectives, technical solutions, and advantages of the embodiments of the present application, the following is a detailed description of the technical solutions in the embodiments. If specific conditions are not stated in the embodiments, conventional conditions or those recommended by manufacturers should be followed. Reagents or instruments not specified with manufacturers are standard products available commercially.

It is noted that, where there is no conflict, the embodiments and features within the embodiments of the present application can be combined with each other. The following provides detailed explanations of the present application with reference to specific embodiments.

Menthae Haplocalycis Herba: Pungent-flavored, cool-natured; meridian affinity: lung and liver. It has the effect of dispersing wind-heat, clearing the head and eyes, clearing the throat and promoting the skin eruption, and soothe the liver regulating qi. It is used for treating wind-heat colds, initial stages of warm diseases, wind-heat headaches, red and teary eyes, swelling and pain in the throat, measles that do not break out, wind rash with itching, liver qi stagnation with chest congestion and pain in the subcostal region.

Bran fried Bombyx Batryticatus: Salty and pungent-flavored, neutral-natured; meridian affinity: liver and lung. It has the effects of extinguishing wind and resolving convulsion, eliminating wind and alleviating pain, transforming phlegm and dissipating nodules. It is used for treating frightened epilepsy and convulsion caused by both acute and chronic infantile convulsions, epilepsy, tetanus; deviation of the mouth and eyes due to wind attacking the meridians pattern; and wind-heat headache, red eyes, sore throat, wind rash with itching, scrofula nodules, and the like.

Gypsum Fibrosum: Pungent-flavored, warm-natured; meridian affinity: liver and spleen.

It has the effects of drying dampness, killing parasites, stopping bleeding, alleviating pain, and removing malignant hyperplasia. It is used for treating scabies, damp sores, trauma bleeding, burns, hemorrhoids, prolapsed anus, and warts. It is also used internally for treating diarrhea and abnormal uterine bleeding.

Anemarrhenae Rhizoma: Bitter and sweet-flavored, cold-natured; meridian affinity: lung and stomach. It has the effects of clearing heat and draining fire, and nourishing yin and moistening dryness. It is used for treating heat-related thirst, lung heat with dry cough, bone steaming tidal fever, internal heat and wasting thirst, and intestinal dryness with constipation.

Scutellariae Radix: Bitter-flavored, cold-natured; meridian affinity: lung, gallbladder, and spleen. It has the effects of clearing heat and drying dampness, draining fire and detoxifying, stopping bleeding, and quieting the fetus. It is used for treating damp-heat, summer heat, chest congestion with nausea, damp-heat gastric fullness, jaundice and diarrhea, lung heat cough, high fever with thirst, blood heat with vomiting or bleeding, and threatened abortion.

Artemisiae Annuae Herba: Bitter and pungent-flavored, cold-natured; meridian affinity: liver and gallbladder. It has the effects of clearing and removing deficiency heat, cooling the blood and relieving bone-steaming sensation, clearing summer heat, and stopping malaria. It is used for treating pathogenic warmth damaging yin, night fever abating at dawn, yin deficiency leading to fever, heat from labor or bone steaming tidal fever, summer heat and external contraction, fever and thirst, and malaria cold-heat.

Scrophulariae Radix: Sweet, bitter, and salty-flavored; slightly cold-natured. It has the effects of clearing heat and cooling the blood, draining fire and detoxifying, and nourishing yin. It is used for treating symptoms such as heat entering the nutrient level, internal invasion of the pericardium, temperature toxins causing rashes, heat injury to yin, fluid damage with constipation, bone steaming tidal fever with labor cough, red eyes, sore throat, scrofula, diphtheria, and boils and abscesses and sore poison.

Lycii Cortex: Sweet-flavored, cold-natured; meridian affinity: lung, liver, and kidney. It has the effects of cooling the blood and relieving bone-steaming sensation, and clearing the lung and reducing fire. It is used for treating yin deficiency with fever, night sweats, bone steaming tidal fever, lung heat cough, and blood heat bleeding.

Arctii Fructus: having the effects of dispersing wind-heat, dispersing the lungs and resolving phlegm, clearing the throat and promoting the skin eruption, and removing toxins and resolving swelling. It is used for treating wind-heat colds, initial stages of warm diseases, measles that do not break out, wind rash with itching, boils and abscesses and sore poison, erysipelas, and mumps and throat paralysis.

Phragmitis Rhizoma: having the effects of clearing heat and draining fire, generating fluids and alleviating thirst, alleviating vexation, stopping vomiting, and promoting urination. It is used for treating heat-related thirst, stomach heat with vomiting, lung heat cough, lung abscess with purulent expectoration, and heat strangury with pain.

The present application provides a traditional Chinese medicine composition for treating pediatric exogenous wind-heat, which comprises the following raw materials: a Menthae Haplocalycis Herba, a Bombyx Batryticatus, a Gypsum Fibrosum, an Anemarrhenae Rhizoma, a Scutellariae Radix, an Artemisiae Annuae Herba, a Scrophulariae Radix, and a Lycii Cortex.

The formulation of the present application uses the Menthae Haplocalycis Herba, the Bombyx Batryticatus, and the Artemisiae Annuae Herba which are clear and refreshing flavored, gently cooling and dispersing, and are effective in releasing Wei-defence phase pattern; as well as the Gypsum Fibrosum, the Anemarrhenae Rhizoma, and the Scutellariae Radix, which are bitter and cold flavored, and cold and cool-natured, and are effective in clearing heat from the qi level; added with Scrophulariae Radix and the Lycii Cortex, which help nourishing yin and generating fluids, draining fire and detoxifying, preventing heat from damaging yin, and have the purpose of "for every measure of body fluids preserved, there is a measure of vitality maintained". In this way, external wind is dispersed, heat and toxins are cleared, yin fluids are preserved, and fever is rapidly reduced to aid in the pediatric patient's recovery. The combination of these components have the effect of dispersing wind and dissipating heat, clearing heat and detoxifying, generating fluids and protecting yin, function in treating the pattern involving both Wei-defence and qi phases, and treating both the surface and interior conditions, and are primarily used to removing wind and clearing heat, and detoxifying and protecting yin, so as to treating pediatric exogenous wind-heat and the pattern involving both Wei-defence and qi phases, with symptoms such as persistent high fever, sweating or scant sweating, nasal congestion, runny nose, red and swollen throat, yellow urine, dry stool, red tongue with thin white or thin yellow coating, and slippery rapid pulse, and pediatric upper respiratory tract infections with the above syndromes.

In some embodiments of the present application, the traditional Chinese medicine composition comprises the following raw materials in parts by mass: 3 to 11 parts of the Menthae Haplocalycis Herba, 3 to 11 parts of the Bombyx Batryticatus, 15 to 25 parts of the Gypsum Fibrosum, 7 to 17 parts of the Anemarrhenae Rhizoma, 7 to 17 parts of the Scutellariae Radix, 7 to 17 parts of the Artemisiae Annuae Herba, 7 to 17 parts of the Scrophulariae Radix, and 7 to 17 parts of Lycii Cortex. Under the above formulation, the resulting traditional Chinese medicine composition has optimal effect in treating pediatric wind-heat cold disorder.

In other embodiments of the present application, the traditional Chinese medicine composition comprises the following raw materials in parts by mass: 5 to 9 parts of the Menthae Haplocalycis Herba, 5 to 9 parts of the Bombyx Batryticatus, 18 to 24 parts of the Gypsum Fibrosum, 10 to 14 parts of the Anemarrhenae Rhizoma, 10 to 14 parts of the Scutellariae Radix, 10 to 14 parts of the Artemisiae Annuae Herba, 10 to 14 parts of the Scrophulariae Radix, and 10 to 14 parts of Lycii Cortex.

In some embodiments of the present application, the Bombyx Batryticatus is specifically a bran fried Bombyx Batryticatus. Regular Bombyx Batryticatus has a relative strong pungent dissipating power, and the efficacy is strong. While bran fried Bombyx Batryticatus slightly reduce the efficacy of removing wind and releasing the exterior and is better at transforming phlegm and dissipating nodules. Combined with Menthae Haplocalycis Herba and Artemisiae Annuae Herba, bran fried Bombyx Batryticatus is more effective in gently cooling and dispersing, and releasing Wei-defence phase pattern;

Embodiments of the present application provide a preparation method for the traditional Chinese medicine composition for treating the pediatric exogenous wind-heat. The preparation method comprises the following steps:
collecting and mixing the raw materials to obtain a first mixture;
submerging the first mixture in water, soaking and decocting the first mixture, and filtering to obtain a primary filtrate and a filtration residue; mixing the filtration residue with water, decocting, and filtering to obtain a secondary filtrate; and combining the primary filtrate and the secondary filtrate to obtain an extract;
concentrating the extract to obtain a solid extract paste; and
screening the solid extract paste, and mixing a screened solid extract paste with an excipient to form granules, whereby obtaining the traditional Chinese medicine composition for treating the pediatric exogenous wind-heat.

The raw materials are mixed and soaked in water, then decocted twice, such that beneficial substances can be effectively extracted from the raw materials. The extract is then concentrated to obtain a final product. The entire process is simple, convenient, and suitable for bulk production.

In some embodiments of the present application, a mass ratio of the first mixture to water is 1:(10 to 15), the first mixture is soaked in water for 15 mins to 45 mins and decocted for 30 mins to 90 mins. A mass ratio of the filtration residue to water is 1:(6 to10), and the filtration residue a after being mixed with water is decocted for 30 mins to 90 mins. The concentrating is carried out by concentrating the extract at a temperature of less than or equal to 70°C and a vacuum degree of -0.07 MPa to obtain the solid extract paste having a relative density of 1.2-1.3. The excipient is a dextrin, a mass ratio of the solid extract paste to the excipient is 1:3, and the granules are screened by a 14-mesh sieve.

In some embodiments of the present application, a step of mixing the solid extract paste with the dextrin is performed by heating a dried inlet air to a temperature of 60°C to 70°C, introducing a heated air into a fluidized bed from a bottom of the fluidized bed for preheating for 30 mins, and the solid extract paste is enabled to pass through a 100-mesh sieve and then mixed with the dextrin to obtain the traditional Chinese medicine composition in a powder form.

The traditional Chinese medicine composition in the powder form is driven by the heated air to form a fluidized state, and water is atomized into droplets, which are distributed in a bed layer of the fluidized bed and collided with the traditional Chinese medicine composition in the powder form, whereby forming granules; air is continued to be introduced for 5 mins to 30 mins, the granules are screened by a 14-mesh sieve, whereby yielding the traditional Chinese medicine composition for treating the pediatric exogenous wind-heat; and

In some embodiments of the present application, a mass ratio of water to the powder is 1:3, a water flow rate is 5.4 g/min/kg to 21.6 g/min/kg, a water temperature is 25°C to 55°C, and a water atomization pressure is 1 bar to 2 bar, and the traditional Chinese medicine composition for treating the pediatric exogenous wind-heat has a water content of less than 9 wt. %.

The application also provides a preparation method for the traditional Chinese medicine composition for treating the pediatric exogenous wind-heat comprises the following steps:
collecting and mixing the raw materials to obtain a second mixture;
submerging the second mixture in water, heating until reaching a boiling state, and decocting the second mixture at the boiling state, then filtering to obtain a decoction liquid and a decoction residue; mixing the decoction residue with water, heating and refluxing to obtain a reflux liquid, and mixing the decoction liquid with the reflux liquid, concentrating a resulting mixed liquid to obtain a concentrated liquid; and
spray drying the concentrated liquid and mixing the same with an excipient to form granules , and screening to obtain the traditional Chinese medicine composition for treating the pediatric exogenous wind-heat.

The raw materials are soaked in water, then heated to the boiling state and decocted, so as to extract beneficial substances therefrom. After that the decoction residue is refluxed so as to further extract the beneficial substance. In this way, it is ensured that a thorough extraction is performed on the raw materials. Finally, the final spray-drying at low temperatures preserves beneficial substances while achieving thorough drying, thus forming the final product.

In some embodiments of the present application, a mass ratio of the second mixture to water is 1 :(10 to15), and the second mixture after being mixed with the water is decocted in the boiling state for 30 mins to 90 mins. A mass ratio of the decoction residue to water is 1:(6 to 10), and the refluxing is performed for 30 mins to 90 mins; the concentrating is performed for 2 hrs to 3 hrs, and a relative density of the concentrated liquid is 1.2 to 1.3.

In some embodiments of the present application, the spray drying is performed at a temperature of the inlet air being less than 70°C and a feed flow rate being1 kg/min to 1.6 kg/min. The excipient is dextrin. A mass ratio of the concentrated liquid after being spray dried to the excipient is 1:3; and the granules are screened by a 14-mesh sieve.

Detailed description are further made hereinbelow in conjunction with examples to further illustrate the features and performance of the application.

### Example 1

A traditional Chinese medicine composition for treating pediatric exogenous wind-heat was prepared by the following preparation steps:
13 g of a Menthae Haplocalycis Herba, 3 g of a Bombyx Batryticatus, 15 g of a Gypsum Fibrosum, 5 g of an Anemarrhenae Rhizoma, 10 g of a Scutellariae Radix, 7 g of an Artemisiae Annuae Herba, 7 g of an Scrophulariae Radix, and 7 g of a Lycii Cortex were provided as raw materials.

The above raw materials were collected and mixed to obtain a first mixture.

The first mixture was submerged in water according to a mass ratio of the first mixture to the water being 1: 10. The first mixture was soaked for 15 mins and decocted for 30 mins. Then, filtration was performed to obtain a primary filtrate and a filtration residue. Thereafter, the filtration residue was mixed with water according to a mass ratio of the filtration residue to water being 1:6, and decocted for 30 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.2.

An inlet air was dried by the rotary dehumidifier. The dried inlet air was introduced to pass through a primary filter, heated by a heater to a temperature of 60°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 1 bar compressed air and dispersed in the fluidized bed at a speed of 5.4 g/min/kg and collided with the traditional Chinese medicine composition in the powder form to form granules. Air was continued being introduced for 5 mins, the formed granules were screened by a 14-mesh sieve to obtain a traditional Chinese medicine composition for treating pediatric exogenous wind-heat with a water content less than 9 wt. %.

### Example 2

A traditional Chinese medicine composition for treating pediatric exogenous wind-heat was prepared by the following preparation steps:
3 g of a Menthae Haplocalycis Herba, 3 g of a Bombyx Batryticatus, 15 g of a Gypsum Fibrosum, 7 g of an Anemarrhenae Rhizoma, 7 g of a Scutellariae Radix, 7 g of an Artemisiae Annuae Herba, 7 g of an Scrophulariae Radix, and 7 g of a Lycii Cortex were provided as raw materials.

The above raw materials were collected and mixed to obtain a first mixture.

The first mixture was submerged in water according to a mass ratio of the first mixture to the water being 1: 10. The first mixture was soaked for 15 mins and decocted for 30 mins. Then, filtration was performed to obtain a primary filtrate and a filtration residue. Thereafter, the filtration residue was mixed with water according to a mass ratio of the filtration residue to water being 1:6, and decocted for 30 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.2.

An inlet air was dried by the rotary dehumidifier. The dried inlet air was introduced to pass through a primary filter, heated by a heater to a temperature of 60°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 1 bar compressed air and dispersed in the fluidized bed at a speed of 5.4 g/min/kg and collided with the traditional Chinese medicine composition in the powder form to form granules. Air was continued being introduced for 5 mins, the formed granules were screened by a 14-mesh sieve to obtain a traditional Chinese medicine composition for treating pediatric exogenous wind-heat with a water content less than 9 wt. %.

### Example 3

A traditional Chinese medicine composition for treating pediatric exogenous wind-heat was prepared by the following preparation steps:
5 g of a Menthae Haplocalycis Herba, 5 g of a Bombyx Batryticatus, 18 g of a Gypsum Fibrosum, 10 g of an Anemarrhenae Rhizoma, 10 g of a Scutellariae Radix, 10 g of an Artemisiae Annuae Herba, 10 g of an Scrophulariae Radix, and 10 g of a Lycii Cortex were provided as raw materials.

The above raw materials were collected and mixed to obtain a first mixture.

The first mixture was submerged in water according to a mass ratio of the first mixture to the water being 1: 13. The first mixture was soaked for 30 mins and decocted for 30 mins. Then, filtration was performed to obtain a primary filtrate and a filtration residue. Thereafter, the filtration residue was mixed with water according to a mass ratio of the filtration residue to water being 1:8, and decocted for 60 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.25.

An inlet air was dried by the rotary dehumidifier. The dried inlet air was introduced to pass through a primary filter, heated by a heater to a temperature of 65°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 1.5 bar compressed air and dispersed in the fluidized bed at a speed of 13 g/min/kg and collided with the traditional Chinese medicine composition in the powder form to form granules. Air was continued being introduced for 15 mins, the formed granules were screened by a 14-mesh sieve to obtain a traditional Chinese medicine composition for treating pediatric exogenous wind-heat with a water content less than 9 wt. %.

### Example 4

A traditional Chinese medicine composition for treating pediatric exogenous wind-heat was prepared by the following preparation steps:
7 g of a Menthae Haplocalycis Herba, 7 g of a Bombyx Batryticatus, 21 g of a Gypsum Fibrosum, 12 g of an Anemarrhenae Rhizoma, 12 g of a Scutellariae Radix, 12 g of an Artemisiae Annuae Herba, 12 g of an Scrophulariae Radix, and 12 g of a Lycii Cortex were provided as raw materials.

The above raw materials were collected and mixed to obtain a first mixture.

The first mixture was submerged in water according to a mass ratio of the first mixture to the water being 1: 13. The first mixture was soaked for 30 mins and decocted for 30 mins. Then, filtration was performed to obtain a primary filtrate and a filtration residue. Thereafter, the filtration residue was mixed with water according to a mass ratio of the filtration residue to water being 1:8, and decocted for 60 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.25.

An inlet air was dried by the rotary dehumidifier. The dried inlet air was introduced to pass through a primary filter, heated by a heater to a temperature of 65°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 1.5 bar compressed air and dispersed in the fluidized bed at a speed of 13 g/min/kg and collided with the traditional Chinese medicine composition in the powder form to form granules. Air was continued being introduced for 15 mins, the formed granules were screened by a 14-mesh sieve to obtain a traditional Chinese medicine composition for treating pediatric exogenous wind-heat with a water content less than 9 wt. %.

### Example 5

A traditional Chinese medicine composition for treating pediatric exogenous wind-heat was prepared by the following preparation steps:
9 g of a Menthae Haplocalycis Herba, 9 g of a Bombyx Batryticatus, 24 g of a Gypsum Fibrosum, 14 g of an Anemarrhenae Rhizoma, 14 g of a Scutellariae Radix, 14 g of an Artemisiae Annuae Herba, 14 g of an Scrophulariae Radix, and 14 g of a Lycii Cortex were provided as raw materials.

The above raw materials were collected and mixed to obtain a first mixture.

The first mixture was submerged in water according to a mass ratio of the first mixture to the water being 1: 13. The first mixture was soaked for 30 mins and decocted for 30 mins. Then, filtration was performed to obtain a primary filtrate and a filtration residue. Thereafter, the filtration residue was mixed with water according to a mass ratio of the filtration residue to water being 1:8, and decocted for 60 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.25.

An inlet air was dried by the rotary dehumidifier. The dried inlet air was introduced to pass through a primary filter, heated by a heater to a temperature of 65°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 1.5 bar compressed air and dispersed in the fluidized bed at a speed of 13 g/min/kg and collided with the traditional Chinese medicine composition in the powder form to form granules. Air was continued being introduced for 15 mins, the formed granules were screened by a 14-mesh sieve to obtain a traditional Chinese medicine composition for treating pediatric exogenous wind-heat with a water content less than 9 wt. %.

### Example 6

A traditional Chinese medicine composition for treating pediatric exogenous wind-heat was prepared by the following preparation steps:
11 g of a Menthae Haplocalycis Herba, 11 g of a Bombyx Batryticatus, 25 g of a Gypsum Fibrosum, 17 g of an Anemarrhenae Rhizoma, 17 g of a Scutellariae Radix, 17 g of an Artemisiae Annuae Herba, 17 g of an Scrophulariae Radix, and 17 g of a Lycii Cortex were provided as raw materials.

The above raw materials were collected and mixed to obtain a first mixture.

The first mixture was submerged in water according to a mass ratio of the first mixture to the water being 1:15. The first mixture was soaked for 45 mins and decocted for 90 mins. Then, filtration was performed to obtain a primary filtrate and a filtration residue. Thereafter, the filtration residue was mixed with water according to a mass ratio of the filtration residue to water being 1:10, and decocted for 90 mins. Another filtration was performed to obtain a secondary filtrate. After that, the primary filtrate and the secondary filtrate were combined to obtain an extract.

The extract was concentrated at a temperature of lower than or equal to 70°C and a vacuum degree of -0.07 MPa to obtain a solid extract paste having a relative density of 1.3.

An inlet air was dried by the rotary dehumidifier. The dried inlet air was introduced to pass through a primary filter, heated by a heater to a temperature of 70°C, and then introduced into a fluidized bed from a bottom of the fluidized bed where the inlet air was preheated for 30 mins. Thereafter, the solid extract paste was dried, and then screened by a 100-mesh sieve. A screened solid extract paste was mixed with an excipient (dextrin) at a mass ratio of 1:3 to obtain a traditional Chinese medicine composition in a powder form.

The traditional Chinese medicine composition in the powder form was fluidized under the driving of the heated air. Water was added at a mass ratio of 1:3 to the traditional Chinese medicine composition in the powder form, and a peristaltic pump was adopted to transport the water to a spray gun pipe, where the water was atomized into small droplets by 2 bar compressed air and dispersed in the fluidized bed at a speed of 21.6 g/min/kg and collided with the traditional Chinese medicine composition in the powder form to form granules. Air was continued being introduced for 30 mins, the formed granules were screened by a 14-mesh sieve to obtain a traditional Chinese medicine composition for treating pediatric exogenous wind-heat with a water content less than 9 wt. %.

### Example 7

This example was basically similar to Example 3, except that the preparation method was different.

A traditional Chinese medicine composition for treating pediatric exogenous wind-heat was prepared by the following preparation steps:
5 g of a Menthae Haplocalycis Herba, 5 g of a Bombyx Batryticatus, 18 g of a Gypsum Fibrosum, 10 g of an Anemarrhenae Rhizoma, 10 g of a Scutellariae Radix, 10 g of an Artemisiae Annuae Herba, 10 g of an Scrophulariae Radix, and 10 g of a Lycii Cortex were provided as raw materials.

The above raw materials were collected and mixed to obtain a second mixture.

The second mixture was submerged in water according to a mass ratio of the second mixture to water being 1: 10, heated to reach a boiling state, and decocted at the boiling state for 30 mins. Then filtration was performed to obtain a decoction liquid and a decoction residue. The decoction residue was mixed with water according to a mass ratio of the decoction residue to water being 1:6, then heated to a reflux state, which state was maintained for 30 mins, so as to obtain a reflux liquid. The decoction liquid was mixed with the reflux liquid, and a resulting mixed liquid was concentrated for 2 hrs to obtain a concentrated liquid having a relative density of 1.2.

The concentrated liquid was spray dried at a temperature of the inlet air being less than 70°C and a feed flow rate being1 kg/min, then mixed with an excipient at a mass ratio of 1:3, a resulting product was screened by a 14-mesh sieve to obtain the traditional Chinese medicine composition for treating the pediatric exogenous wind-heat.

### Example 8

This example was basically similar to Example 3, except that the preparation method was different.

A traditional Chinese medicine composition for treating pediatric exogenous wind-heat was prepared by the following preparation steps:
7 g of a Menthae Haplocalycis Herba, 7 g of a Bombyx Batryticatus, 21 g of a Gypsum Fibrosum, 12 g of an Anemarrhenae Rhizoma, 12 g of a Scutellariae Radix, 12 g of an Artemisiae Annuae Herba, 12 g of an Scrophulariae Radix, and 12 g of a Lycii Cortex were provided as raw materials.

The above raw materials were collected and mixed to obtain a second mixture.

The second mixture was submerged in water according to a mass ratio of the second mixture to water being 1: 13, heated to reach a boiling state, and decocted at the boiling state for 60 mins. Then filtration was performed to obtain a decoction liquid and a decoction residue. The decoction residue was mixed with water according to a mass ratio of the decoction residue to water being 1:8, then heated to a reflux state, which state was maintained for 60 mins, so as to obtain a reflux liquid. The decoction liquid was mixed with the reflux liquid, and a resulting mixed liquid was concentrated for 2.5 hrs to obtain a concentrated liquid having a relative density of 1.25.

The concentrated liquid was spray dried at a temperature of the inlet air being less than 70°C and a feed flow rate being 1.3 kg/min, then mixed with an excipient at a mass ratio of 1:3, a resulting product was screened by a 14-mesh sieve to obtain the traditional Chinese medicine composition for treating the pediatric exogenous wind-heat.

### Example 9

This example was basically similar to Example 5, except that the preparation method was different.

A traditional Chinese medicine composition for treating pediatric exogenous wind-heat was prepared by the following preparation steps:
9 g of a Menthae Haplocalycis Herba, 9 g of a Bombyx Batryticatus, 24 g of a Gypsum Fibrosum, 14 g of an Anemarrhenae Rhizoma, 14 g of a Scutellariae Radix, 14 g of an Artemisiae Annuae Herba, 14 g of an Scrophulariae Radix, and 14 g of a Lycii Cortex were provided as raw materials.

The above raw materials were collected and mixed to obtain a second mixture.

The second mixture was submerged in water according to a mass ratio of the second mixture to water being 1: 15, heated to reach a boiling state, and decocted at the boiling state for 90 mins. Then filtration was performed to obtain a decoction liquid and a decoction residue. The decoction residue was mixed with water according to a mass ratio of the decoction residue to water being 1: 10, then heated to a reflux state, which state was maintained for 90 mins, so as to obtain a reflux liquid. The decoction liquid was mixed with the reflux liquid, and a resulting mixed liquid was concentrated for 3 hrs to obtain a concentrated liquid having a relative density of 1.3.

The concentrated liquid was spray dried at a temperature of the inlet air being less than 70°C and a feed flow rate being 1.6 kg/min, then mixed with an excipient at a mass ratio of 1:3, a resulting product was screened by a 14-mesh sieve to obtain the traditional Chinese medicine composition for treating the pediatric exogenous wind-heat.

### Comparative Example 1

This example was basically similar to Example 4 except that Scrophulariae Radix was replaced by Phragmitis Rhizoma.

### Comparative Example 2

This example was basically similar to Example 4 except that Menthae Haplocalycis Herba was replaced by Arctii Fructus.

### Clinical Trial

(1) Data Collection: 168 pediatric patients were randomly divided into 4 groups, in which, a treatment Group (n=42) included 23 males and 19 females, with ages from 3 years to 10 years and a disease duration of 14 hrs to 36 hrs; a control Group 1 (n=42) included 20 males and 22 females, with ages from 3 years to 7 years and a disease duration of 15 hrs to 37 hrs; a control Group 2 (n=42) included 21 males and 21 females, with ages from 3 years to 8 years and a disease duration 14-37 hrs; and a control Group 3 (n=42) included 19 males and23 females, with ages from 3 years to 9 years and a disease duration 15-37 hrs. The general data of the four groups of pediatric patients, including sex, age, and disease duration, were statistically analyzed, which showed that the differences (P > 0.05) were not statistically significant, indicating comparability among the groups;
(2) Diagnostic criteria: the diagnostic criteria for cold wind-heat attacking the exterior syndrome is referred to the pediatric section of the "Standards for Diagnosis and Efficacy of TCM Diseases and Syndromes", and includes symptoms including: severe fever, mild aversion to cold, sweating or no sweating, headache, nasal congestion and thick runny nose, cough, red throat, or red eyes and tears, irritability and thirst. The tongue is red with little fluid, the tongue coating is thin and yellow, and the pulse is floating and rapid. Clinical manifestations include fever, nasal congestion, runny nose, sore throat, diarrhea, headache, fatigue, etc.
(3) Treatment method:
   Pediatric patients of a control group 1 took the commercially available Xiaoer Chiqiao Qingre Granules (Jichuang Pharmaceutical Group Co., Ltd., product batch number 2203094, specification 2g*6 bags/box) with warm water; pediatric patients of control group 2 were took the traditional Chinese medicine composition prepared in Comparative Example 1 for treatment; pediatric patients of control group 3 patients took the traditional Chinese medicine composition prepared in Comparative Example 1 for treatment; and pediatric patients of treatment group took the traditional Chinese medicine composition prepared by Jiuhua Huayuan Pharmaceutical Co., Ltd. (prepared in Example 4) for treatment. The above Chinese medicine compositions were taken after meals every day. The pediatric patients were avoided from cold during treatment, kept a regular work and rest schedule, and eaten a light diet. Pediatric patients under 3 years old, 2 g/time, 3 times/d; and pediatric patients aged 3 years to 7 years, 3 g/time, 3 times/d; one week was one course of treatment, and the pediatric patients' recovery was observed every day.
(4) Evaluation indicators: the treatment conditions of the four groups of pediatric patients were observed, and the disappearance time of fever, cough, sore throat, and nasal congestion and runny nose of the four groups of pediatric patients was recorded. Meanwhile, the occurrences of adverse reactions such as nausea, vomiting, and diarrhea during the medication period of the two groups of pediatric patients were observed. The treatment conditions are shown in Table 1.
(5) Data processing method: SPSS 22.0 software was used to analyze the data. The measurement data were expressed as x±s and the t test was used. The count data were expressed as percentages and the x^2 test was used. P<0.05 indicated that the difference was statistically significant.
6) Results: The total effective rate of control group 1 was 83.33% (35/42), the total effective rate of control group 2 was 85.71% (36/42), the total effective rate of control group 3 was 83.33% (35/42), and the total effective rate of the treatment group was 95.24% (40/42). The difference among the four groups was statistically significant (P<0.05), as shown in Table 2;

**Table 1 Comparison of the time to resolution of clinical symptoms and signs between the two groups (x̅±s, d)**

| Group | Number of cases | Fever Resolution Time | Cough Resolution Time | Throat Redness and Swelling Resolution Time | Nasal Congestion and Runny Nose Resolution Time |
|---|---|---|---|---|---|
| Control Group 1 | 42 | 4.03±0.54 | 5.94±0.61 | 4.37±0.46 | 4.08±0.65 |
| Control Group 2 | 42 | 4.02±0.41 | 4.29±0.56 | 4.21±0.53 | 4.18±0.42 |
| Control Group 3 | 42 | 3.95±0.39 | 4.09±0.35 | 4.18±0.48 | 4.22±0.51 |
| Treatment Group | 42 | 3.65±0.38* | 3.43±0.41** | 2.03±0.44* | 2.58±0.54** |

| | | | | | |
|---|---|---|---|---|---|
| Note: Compared to the control group, *P<0.05, **P<0.01. | | | | | |

**Table 2 Comparison of Clinical Efficacy Between Two Groups Cases (%)**

| Group | Number of Cases | Cured | Significantly Effective | Effective | Ineffective | Total Effective |
|---|---|---|---|---|---|---|
| Control Group 1 | 42 | 12 (28.57) | 13 (30.95) | 10 (23.81) | 7 (16.67) | 35 (83.33) |
| Control Group 2 | 42 | 12 (28.57) | 13 (30.95) | 11 (23.81) | 6 (14.29) | 36 (85.71) |
| Control Group 3 | 42 | 12 (28.57) | 11 (26.19) | 12 (28.57) | 7 (16.67) | 35 (83.33) |
| Treatment Group | 42 | 13 (30.95) | 15 (35.71) | 12 (28.57) | 2 (4.76) | 40 (95.24) |

| | | | | | | |
|---|---|---|---|---|---|---|
| Note: Compared to the control group, *P<0.05. | | | | | | |

As shown in Table 1, compared with the control groups 1-3, the treatment group had obvious effects on reducing fever, cough, sore throat, and nasal congestion and runny nose, and had obvious therapeutic effects on cough, sore throat, and nasal congestion and runny nose.

It was known from Table 2 that the treatment group only had 2 cases that were ineffective to the treatment, and the control group 1 had 12 cases that were ineffective to the treatment; and the cured, significantly effective, effective, and total effective rate of the treatment group were better than those of the control groups. When comparing the treatment group and the control groups 2-3, Scrophulariae Radix and Phragmitis Rhizoma both had the effect of clearing heat and draining fire, Arctii Fructus and Menthae Haplocalycis Herba both had the effect of dispersing wind and dissipating heat, and the therapeutic effect of the traditional Chinese medicine composition obtained by replacing the medicinal materials with similar efficacy was not as good as before the replacement. It can be seen that replacing the raw materials in the formula of the present application with medicinal materials with similar efficacy will affect the efficacy, and the compatibility scheme of the present application is feasible and has outstanding effects.

In conclusion, the researchers of the present application believe that pediatric upper respiratory infections fall under the category of "colds" or "wind-cold" in traditional Chinese medicine (TCM). Since the lung governs the skin and the pores, and children's organs are delicate with weak lung defense functions, when they are exposed to the wind-heat, or the wind-cold enters the interior and transforms into heat, then the defensive surface is out of harmony and the lung qi fail to disperse properly. This leads to symptoms of defensive surface and the upper jiao of pulmonary system, such as fever, nasal congestion, runny nose, cough, and headache.

Based on the above reasons, the present application uses the Menthae Haplocalycis Herba, the Bombyx Batryticatus, and the Artemisiae Annuae Herba which are clear and refreshing flavored, gently cooling and dispersing, and are effective in releasing Wei-defence phase pattern; as well as the Gypsum Fibrosum, the Anemarrhenae Rhizoma, and the Scutellariae Radix, which are bitter and cold flavored, and cold and cool-natured, and are effective in clearing heat from the qi level; added with Scrophulariae Radix and the Lycii Cortex, which help nourishing yin and generating fluids, draining fire and detoxifying, preventing heat from damaging yin, and have the purpose of "for every measure of body fluids preserved, there is a measure of vitality maintained". In this way, external wind is dispersed, heat and toxins are cleared, yin fluids are preserved, and fever is rapidly reduced to aid in the pediatric patient's recovery. The combination of these components have the effect of dispersing wind and dissipating heat, clearing heat and detoxifying, generating fluids and protecting yin, function in treating the pattern involving both Wei-defence and qi phases, and treating both the surface and interior conditions, and are primarily used to removing wind and clearing heat, and detoxifying and protecting yin, so as to treating pediatric exogenous wind-heat and the pattern involving both Wei-defence and qi phases, with symptoms such as persistent high fever, sweating or scant sweating, nasal congestion, runny nose, red and swollen throat, yellow urine, dry stool, red tongue with thin white or thin yellow coating, and slippery rapid pulse, and pediatric upper respiratory tract infections with the above syndromes.

In conclusion, the traditional Chinese medicine composition provided in the present application can effectively treat pediatric wind-heat exterior conditions, significantly improve clinical symptoms and signs in pediatric patient, enhance immune function, and has a notable therapeutic effect with high safety, making it suitable for clinical promotion.

The present application also provides two preparation methods for the traditional Chinese medicine composition, which maximize the extraction of beneficial substances from the ingredients and efficiently and conveniently produce the traditional Chinese medicine composition, which facilitates the bulk production.

The embodiments described in the above are part of, rather than all of, the embodiments of the present application. The detailed description of the embodiments of the present application is not intended to limit the scope of the claims, but merely to illustrate optional embodiments of the present application. All other embodiments obtained by those skilled in the art without making inventive efforts based on the embodiments in the present application fall within the scope of protection of the present application.

## Claims

1. A traditional Chinese medicine composition for treating pediatric exogenous wind-heat, **characterized by** comprising the following raw materials:
a Menthae Haplocalycis Herba,
a Bombyx Batryticatus,
a Gypsum Fibrosum,
an Anemarrhenae Rhizoma,
a Scutellariae Radix,
an Artemisiae Annuae Herba,
a Scrophulariae Radix, and
a Lycii Cortex.

2. The traditional Chinese medicine composition for treating the pediatric exogenous wind-heat according to claim 1, wherein the traditional Chinese medicine composition comprises the following raw materials in parts by mass:
3 to 11 parts of the Menthae Haplocalycis Herba,
3 to 11 parts of the Bombyx Batryticatus,
15 to 25 parts of the Gypsum Fibrosum,
7 to 17 parts of the Anemarrhenae Rhizoma,
7 to 17 parts of the Scutellariae Radix,
7 to 17 parts of the Artemisiae Annuae Herba,
7 to 17 parts of the Scrophulariae Radix, and
7 to 17 parts of Lycii Cortex.

3. The traditional Chinese medicine composition for treating the pediatric exogenous wind-heat according to claim 1 or 2, wherein the traditional Chinese medicine composition comprises the following raw materials in parts by mass:
5 to 9 parts of the Menthae Haplocalycis Herba,
5 to 9 parts of the Bombyx Batryticatus,
18 to 24 parts of the Gypsum Fibrosum,
10 to 14 parts of the Anemarrhenae Rhizoma,
10 to 14 parts of the Scutellariae Radix,
10 to 14 parts of the Artemisiae Annuae Herba,
10 to 14 parts of the Scrophulariae Radix, and
10 to 14 parts of Lycii Cortex.

4. The traditional Chinese medicine composition for treating the pediatric exogenous wind-heat according to claim 1, wherein the Bombyx Batryticatus is bran fried Bombyx Batryticatus.

5. A preparation method for the traditional Chinese medicine composition for treating the pediatric exogenous wind-heat according to any one of claims 1-4, **characterized by** comprising the following steps:
collecting and mixing the raw materials to obtain a first mixture;
submerging the first mixture in water, soaking and decocting the first mixture, and filtering to obtain a primary filtrate and a filtration residue; mixing the filtration residue with water, decocting, and filtering to obtain a secondary filtrate; and combining the primary filtrate and the secondary filtrate to obtain an extract;
concentrating the extract to obtain a solid extract paste; and
screening the solid extract paste, and mixing a screened solid extract paste with an excipient to form granules, whereby obtaining the traditional Chinese medicine composition for treating the pediatric exogenous wind-heat.

6. The preparation method according to claim 5, wherein
a mass ratio of the first mixture to water is 1 :(10 to 15), the first mixture is soaked in water for 15 mins to 45 mins and decocted for 30 mins to 90 mins;
a mass ratio of the filtration residue to water is 1:(6 to10), and the filtration residue a after being mixed with water is decocted for 30 mins to 90 mins;
the concentrating is carried out by concentrating the extract at a temperature of less than or equal to 70°C and a vacuum degree of -0.07 MPa to obtain the solid extract paste having a relative density of 1.2-1.3; and
the excipient is a dextrin, a mass ratio of the solid extract paste to the excipient is 1:3, and the granules are screened by a 14-mesh sieve.

7. The preparation method according to claim 5, wherein
a step of mixing the solid extract paste with the dextrin is performed by heating a dried inlet air to a temperature of 60°C to 70°C, introducing a heated air into a fluidized bed from a bottom of the fluidized bed for preheating for 30 mins, and the solid extract paste is enabled to pass through a 100-mesh sieve and then mixed with the dextrin to obtain the traditional Chinese medicine composition in a powder form;
the traditional Chinese medicine composition in the powder form is driven by the heated air to form a fluidized state, and water is atomized into droplets, which are distributed in a bed layer of the fluidized bed and collided with the traditional Chinese medicine composition in the powder form, whereby forming granules; air is continued to be introduced for 5 mins to 30 mins, the granules are screened by a 14-mesh sieve, whereby yielding the traditional Chinese medicine composition for treating the pediatric exogenous wind-heat; and
a mass ratio of water to the powder is 1:3, a water flow rate is 5.4 g/min/kg to 21.6 g/min/kg, a water temperature is 25°C to 55°C, and a water atomization pressure is 1 bar to 2 bar, and the traditional Chinese medicine composition for treating the pediatric exogenous wind-heat has a water content of less than 9 wt. %.

8. A preparation method for the traditional Chinese medicine composition for treating the pediatric exogenous wind-heat according to any one of claims 1-4, **characterized by** comprising the following steps:
collecting and mixing the raw materials to obtain a second mixture;
submerging the second mixture in water, heating until reaching a boiling state, and decocting the second mixture at the boiling state, then filtering to obtain a decoction liquid and a decoction residue; mixing the decoction residue with water, heating and refluxing to obtain a reflux liquid, and mixing the decoction liquid with the reflux liquid, concentrating a resulting mixed liquid to obtain a concentrated liquid; and
spray drying the concentrated liquid and mixing the same with an excipient to form granules , and screening to obtain the traditional Chinese medicine composition for treating the pediatric exogenous wind-heat.

9. The preparation method according to claim 8, wherein
a mass ratio of the second mixture to water is 1:(10 to15), and the second mixture after being mixed with the water is decocted in the boiling state for 30 mins to 90 mins; and
a mass ratio of the decoction residue to water is 1:(6 to 10), and the refluxing is performed for 30 mins to 90 mins; the concentrating is performed for 2 hrs to 3 hrs, and a relative density of the concentrated liquid is 1.2 to 1.3.

10. The preparation method according to claim 8, wherein
the spray drying is performed at a temperature of the inlet air being less than 70°C and a feed flow rate being1 kg/min to 1.6 kg/min;
the excipient is dextrin; and
a mass ratio of the concentrated liquid after being spray dried to the excipient is 1:3; and the granules are screened by a 14-mesh sieve.
